# EUROPEAN PATENT APPLICATION

(11) **EP 1 402 916 A1**
(43) Date of publication of application: **31.03.2004**
(21) Application number: 03015285.4
(22) Date of filing: 07.07.2003
(51) Int. Cl.: A61M 16/18

(54) **Injection vaporiser**

(30) Priority: 25.09.2002 SE 0202832
(71) Applicant: Maquet Critical Care AB, 171 95 Solna (SE)
(72) Inventor: Kock, Mikael, 184 91 Äkersberga (SE)
(74) Representative: Samzelius, Roger Mikael

(57) **Abstract**

An injection vaporiser comprises a reservoir (2) for liquid (4) to be vaporised and a delivery conduit (10) is connected to the reservoir (2) and has an outlet (12) for injecting liquid (4) to be vaporised into a gas space, shown formed by a through-flow chamber (24). A pumping system (8) is operable to transfer liquid from the reservoir (2) to the outlet (12). A heater (14) is also provided and arranged for supplying thermal energy into the delivery conduit (10). A pressure generator (8,20) is configured to generate an elevated delivery pressure within the delivery conduit (10) and is cooperable with the heater (14) to establish a heated pressurised liquid (4) at the outlet (12).

## Description

The present invention relates to an injection vaporiser and in particular to an injection vaporiser for admixing a liquid anaesthetic and a breathing gas.

Injection vaporisers are well known and operate by direct injection of a volatile liquid, such as liquid anaesthetic, into a gas flow, such as a breathing gas flow intended for delivery to a patient. Generally, the known injection vaporisers comprises a pump system connected to a reservoir for liquid to be vaporised and to a delivery conduit. Liquid is transferred into the delivery conduit by the pump system and from there it is injected through an outlet and into the gas flow within a gas space wherein it vaporises. One example of such an injection vaporiser is disclosed in US 5,242,403. Here the disclosed vaporiser is specially adapted for low boiling-point liquids and additionally comprises a cooling device to maintain the temperature of the liquid, particularly within the reservoir and the pumping device, below boiling-point. Since this cooling makes it more difficult for the liquid to vaporise a heating device is provided to supply thermal energy into the delivery conduit to vaporise the liquid therein. However this has the disadvantage that metering of doses of the volatile liquid may become more inaccurate owing to the presence of a mixture of vapour and liquid within the delivery conduit.

According to the present invention there is provided an injection vaporiser as described in and characterised by the present claim 1. By providing a pressure generator that operates in co-operation with a heater in thermal contact with liquid within the delivery conduit then a pressurised heated liquid can be established at the outlet of the delivery tube, preferably heated to a temperature of at least the boiling point of the liquid at a pressure extant external the outlet of the delivery conduit. This aids the rapid volatilisation of the liquid in the gas space into which it is injected and also enables a more accurate metering of the so injected liquid.

Exemplary embodiments of vaporisers according to the present invention will now be described with reference to the drawings of the accompanying figures, of which:
Fig. 1 shows a schematic representation of a first vaporiser according to the present invention;
Fig. 2 shows a schematic representation of a pressure regulator usable in the vaporiser according to the present invention; and
Fig. 3 shows a schematic representation of a second vaporiser according to the present invention;

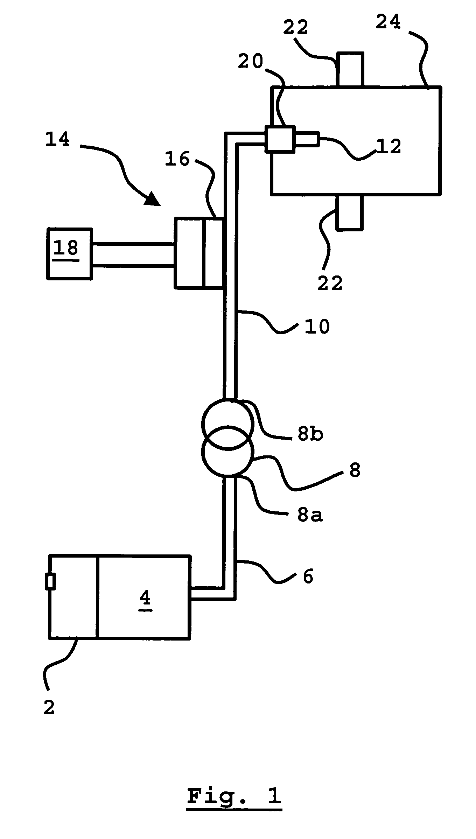

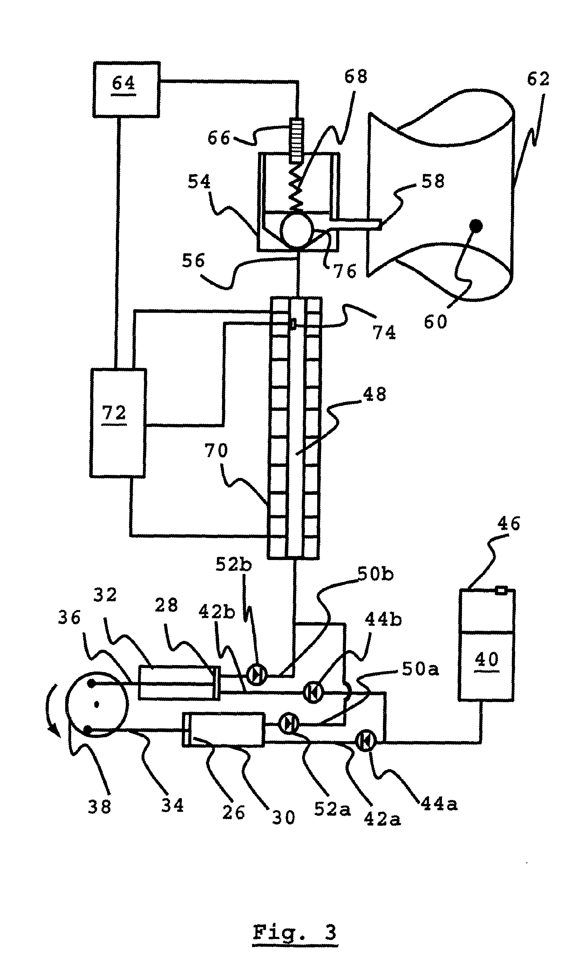

Considering now Fig. 1, a vaporiser comprises a reservoir 2 for liquid 4 to be vaporised connected by a feed conduit 6 to an inlet 8a of a pump system 8 which has an outlet 8b connected to a delivery conduit 10. The delivery conduit 10 has an outlet 12 that extends into a gas space, provided in the present example by a chamber 24, and provides for injection of liquid 4 from the reservoir 2 into the chamber 24. The chamber 24 is, in the present example, provided with gas ports 22 by which a flow of gas to be admixed with vaporised liquid will enter and exit the chamber 24.

A heater 14, such as a Peltier heater, is located with its warming surface 16 in good thermal contact with at least a section of the delivery conduit 10 and is energisable by a power supply 18 to supply thermal energy into the vaporisable liquid within the delivery conduit 10. A pressure regulator 20 is located in fluid communication with the outlet 12 of the delivery conduit 10 and is operable to inhibit injection of liquid 4 into the gas space formed by the chamber 24 until a desired delivery pressure of liquid within the conduit 10 is established.

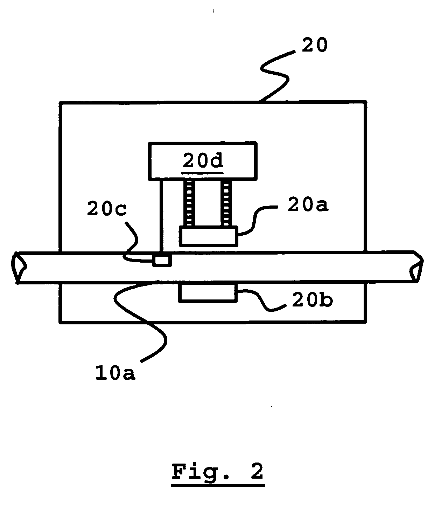

An example of a pressure regulator 20 that may be employed in the vaporiser of Fig. 1 is depicted in Fig. 2. As is illustrated in Fig. 2, the pressure regulator 20 may be formed a so called "pinch" or "clamp" type valve that comprises relatively movable jaws 20a,b between which is located a resiliently deformable wall section 10a of the delivery conduit 10. This wall section 10a may be formed integrally with the delivery conduit 10, as in the present embodiment, or may form a separate conduit section, for example provided as a part of the pressure regulator 20, that is attachable in line with the remainder of the delivery conduit 10. A pressure sensor 20c is provided as part of'the regulator 20 and is arranged to sense pressure within the delivery conduit 10, preferably proximal the jaws 20a,b. The pressure sensor 20c is configured to provide an output to a drive unit 20d, such as a stepper drive, of the regulator 20 which is operably connected to the movable jaws 20a,b so as to be able to move one or both jaws 20a,b in to or out of contact with the deformable conduit section 10a in response to the pressure sensed by the pressure sensor 20c.

Returning now to the exemplary vaporiser of Fig. 1, in use the pumping system 8 is operated to generate a pressurised flow of liquid 4 from the reservoir 2 and towards the outlet 12 of the delivery conduit 10. The pressure regulator 20 is configured to maintain the outlet 12 sealed until the pressure of liquid 4 within the delivery conduit 10, reaches an elevated, predetermined delivery pressure. The heater 14 supplies thermal energy into the delivery conduit 10 to raise the temperature of the liquid therein to a level below its boiling point at the elevated delivery pressure and preferably to a level above its boiling point at ambient pressure within the chamber 24. As the heated pressurised liquid exits through the outlet 12 its pressure is reduced and the supplied thermal energy aids in the volatilisation of the liquid in a gas stream flowing through the chamber 24. The delivery pressure and the maximum temperature to which the heater 14 elevates the liquid 4 are thus clearly inter-related and depend in a known manner on the thermodynamic properties of the liquid 4 to be volatilised.

Considering now the vaporiser of Fig. 2, each one of a pair of pistons 26,28 is arranged for reciprocal displacement within an associated chamber 30,32 to act as a pumping system. Each piston 26,28 is connected via an associated rod 34,36 to a drive 38 in a manner such that, in the present example, their reciprocating pump strokes have a relative phase difference of substantially 180 degrees. The chambers 30,32 act as a reservoir for a liquid anaesthetic 40 to be vaporised and are each connectable via, in the present example, an associated feed line 42a,b and a one-way valve arrangement 44a,b located in respective feed line 42a,b to a supply 46 of the liquid anaesthetic 40. The one-way valve arrangements 44a,b are each configured to permit liquid flow in one direction only, namely from the supply 46 to refill the chambers 30,32 as necessary. Each chamber 30,32 is also connected to a delivery conduit 48 via, in the present example, an associated feed line 50a,b and a one-way valve arrangement 52a,b located in a respective feed line 50a,b and configured to permit liquid flow in one direction only, namely from the chambers 30,32.

A pressure regulator, here in the form of a back pressure valve 54 of a known "ball-and-spring" type, is located in fluid communication with an open end 56 of the delivery conduit 48. The pressure valve 54 is, in the present example, provided with a port 58 that forms the outlet of the delivery conduit 48 and that opens into a gas space 60, here formed by a section 62 of a pneumatic circuit of a mechanical breathing aid (not shown), through which a breathing gas to be delivered to a patient is intended to flow. The pressure valve 54 is configured to seal the outlet port 58 against egress of the liquid anaesthetic 40 until a liquid pressure within the delivery conduit 48, generated by the pistons 26,28, reaches a desired elevated delivery pressure. At this time the pressure valve 54 operates to unseal the outlet port 58 and so permit the injection of heated, pressurised liquid anaesthetic 40 through the port 58 into the gas space 60 where it vaporises. As shown in Fig. 2, the delivery pressure set by the pressure valve 54 is adjustable by means of a controller 64 that here operates a stepper motor 66 to adjust the tension of the spring 68 of the ball-and-spring arrangement. A desired delivery pressure may be input by a user directly into the controller 64 via a user interface such as a keyboard, touch screen or pointing device and graphic display. Alternatively, the liquid anaesthetic 40 to be delivered may be identified by the user to the controller 64 which may then be provided with a look up table resident within an internal memory in which each of one or more liquid anaesthetics is indexed with a desired delivery pressure (being for example a pressure at which the selected anaesthetic remains liquid at a predetermined elevated temperature) to thereby automatically establish a desired delivery pressure.

A heater, here in the form of an elongate wire wound resistance heater 70, is located in thermal contact about at least a portion of the delivery conduit 48 and is energisable by means of a power supply 72 to supply thermal energy into pressurised liquid within the delivery conduit 48. In the present example the power supply 72 is controllable in response to inputs from a thermal sensor 74, within the delivery conduit 48, and also by an output from the pressure valve controller 64, indicating the predetermined temperature to which the liquid anaesthetic within the conduit 48 is to be heated. This predetermined temperature is associated with the elevated delivery pressure to be established by the valve 54 at which the so heated anaesthetic within the conduit 48 will remain liquid but at which the so heated anaesthetic will be proximal, preferably above, its boiling point within the gas space 60. Usefully, the heater 70 may be arranged to also heat the valve 54, and in particular the ball 76 of the ball-and spring arrangement which represents a relatively large heat sink in contact with heated anesthetic 40. This better ensures that the anesthetic 40 present at the port 58 remains heated to about it's boiling point at the pressure within the section 62 of the pneumatic circuit into which the port 58 opens.

In use, and as also described with respect to the vaporiser of Fig. 1, the heated pressurised liquid anaesthetic 40 is injected into the gas space 60, the reduced pressure and the supplied thermal energy aids in the rapid vaporisation of the liquid anaesthetic in the breathing gas within the section 62 of the pneumatic circuit.

## Claims

1. An injection vaporiser comprising a reservoir (2;30,32) for liquid (4;40) to be vaporised; a delivery conduit (10;48) having an outlet (12;58) for injecting liquid (4;40) to be vaporised into a gas space (24;60); a pumping system (8;26,28,34,36,38) operable to transfer liquid from the reservoir (2;30,32) to the outlet (12;58); and a heater (14;70) for supplying thermal energy into the delivery conduit (10;48) **characterised in that** there is also provided a pressure generator (8,20; 26,28,34,36,38,54) for generating an elevated delivery pressure within the delivery conduit (10;48) and co-operable with the heater (14;70) to establish a heated pressurised liquid (4;40) at the outlet (12;58).

2. A vaporiser as claimed in Claim 1 **characterised in that** the pressure generator comprises a pressure regulator (20;54) in fluid communication with the outlet (12;58) and responsive to pressure levels within the delivery conduit (10;48) to inhibit egress of transferred liquid from the outlet (12;58) until the elevated delivery pressure is generated.

3. A vaporiser as claimed in Claim 1 or Claim 2 **characterised in that** the pumping system (8; 26,28,34,36,38) is adapted to also pressurise the liquid (4;40) to generate the elevated delivery pressure.

4. A vaporiser as claimed in any preceding claim **characterised in that** the pumping system comprises a pair of pistons (26;28) each one being mounted for reciprocation within an associated pump chamber (30;32) acting as the reservoir and connected to a drive (38) to operate with pump strokes having a relative phase difference of substantially 180 degrees.

5. A vaporiser as claimed in any preceding claim **characterised in that** the heating device (14;70) is operable to supply thermal energy in an amount to heat the liquid (4;40) within the delivery conduit (10;48) to a temperature of at least the boiling point of the liquid at a pressure external the outlet (12;58).
